# EUROPEAN PATENT SPECIFICATION

(11) **EP 1 083 880 B1**
(45) Date of publication and mention of the grant of the patent: **01.09.2004**
(21) Application number: 99922436.3
(22) Date of filing: 03.06.1999
(51) Int. Cl.: A61K 9/06, A61K 31/565, A61K 31/57

(54) **STABLE GEL MIXTURE IN THE FORM OF A MIXTURE OF OLEOGEL AND AQUEOUS GEL**
STABILGELMISCHUNG IN FORM EINER MISCHUNG EINES ÖLIGEN GELS UND EINES WÄSSRIGEN GELS
MELANGE DE GELS STABLE CONSTITUE D'UN OLEOGEL ET D'UN GEL AQUEUX

(30) Priority: 03.06.1998 FR 9806958; 03.12.1998 US 204089
(43) Date of publication of application: 21.03.2001
(73) Proprietor: Aiache, Jean-Marc, F-63000 Clermont-Ferrand (FR)
(72) Inventor: AIACHE, Jean-Marc, F-63000 Clermont-Ferrand (FR); GAUTHIER, Pascale, F-63730 Les Martres-de-Veyre (FR)
(74) Representative: Ahner, Francis
(86) International application number: PCT/IB1999/001068
(87) International publication number: WO 1999/062497

(56) References cited:
- EP-A- 0 356 325
- EP-A- 0 793 966
- WO-A-93/20799
- J. M. AIACHE, ET AL.: "New gelification method for vegetable oils I: cosmetic application" INT. J. COSMET. SCI., vol. 14, no. 5, 1992, pages 228-234, XP002093985
- AIACHE, J. M.; ET AL.: "New gelification method for vegetal oils" BULL. TECH./GATTEFOSSE REP., vol. 84, 1991, pages 71-76, XP002093986
- P. GAUTHIER; ET AL.: "Novel glyceride gels II. Viscosity characteristics" INT. J. COSMET. SCI., vol. 18, no. 5, October 1996 (1996-10), pages 229-235, XP002093987

## Description

The present invention relates to a stable gel mixture in the form of a mixture of oleogel and aqueous gel, to a process for preparing this stable gel mixture, to a pharmaceutical composition comprising this stable gel mixture, to the use of this composition as a reservoir of active ingredients in a transdermal release system and, lastly, to a cosmetic composition comprising this stable gel mixture.

EP 793 966 is drawn to a topical pharmaceutical composition containing cycloporim as an active ingredient. This composition is in the form of an oil-in-water emulsion comprising a surfactant.

EP 356 325 relates to a pharmaceutical composition comprising an active substance which is poorly soluble by at least a cellulosic gelified glycerid.

Morever it is known to prepare oleogels, in particular by gelling a synthetic, semisynthetic or natural oil, in order to combine the relatively solid consistency of a gel with the total transparency of an oil.

However, oleogels have the drawback of feeling greasy and unpleasant, on account of the presence of the oily compound. This unpleasant feel very often discourages users, in particular in the case of dermatological and cosmetic applications.

It is thus desirable, for example, to be able to give a bodycare oil a relatively solid consistency but with a non-greasy feel, i.e a fresh and pleasant feel, while at the same time keeping the original appearance of the oil. The reason for this is that oils are difficult for users to control because of their excessive fluidity. In particular, the use of bath oils makes the bath slippery and accounts for approximately 3% of the serious accidents in bathrooms.

It has now been found, entirely surprisingly and unexpectedly, that the mixture of a certain type of oleogel with an aqueous gel makes it possible to obtain a gel of substantially uniform appearance, which is remarkably stable and has a fresh and pleasant feel.

Thus the subject of the present invention is a stable gel, that comprises a mixture, of substantially uniform appearance, of at least one oleogel and at least one aqueous gel, the oleogel comprising at least one oily agent gelled with at least one cellulose polymer.

According to the invention, the expression "mixture of substantially uniform appearance" is understood to mean an intimate mixture, and not an emulsion, of the oleo and aqueous gels, that is, a uniform dispersion of one in the other, such that, either only a single gel can be distinguished,when inspected visually, or no separation of the two aqueous gel and oleogel phases is detected, when applied to the skin .

The intimate nature of the mixture of the two types of gel can be controlled, for example, by introducing a dye substance into the oily or aqueous gel, before forming the mixture, in order to visually observe the uniform dispersion of this dye substance after forming the mixture, even though the dye is present in only one of the two types of gel - oily or aqueous - of the stable gel mixture formed.

According to the invention, the term "stable gel" is understood to mean the stable mixture of oily and aqueous gels of a uniform appearance without any demixing on storage.

In particular, the cellulose polymer is chosen from ethylcellulose, non-sodium carboxymethylcellulose, and mixtures thereof.

The oily agent is chosen in particular from mono-, di-, and triglycerides of synthetic, semisynthetic and natural origin, and mixtures thereof.

As synthetic mono-,di- or triglycerides, reference is made in particular to Miglyol 810 and 812, as sold by the company Dynamit Nobel.

As semisynthetic mono-, di- or triglycerides, reference is made in particular to propylene glycol isostearate, such as the product sold under the name "hydrophilol isostéarique" by the company Gatefossé, and the polyglycolysed glyceride "Labrafil® M 1944 Cs" as sold by the company Gatefossé.

Labrafil® M 1944 CS is a mixture of polyoxyethylenated oleic glycerides obtained by alcoholysis of natural plant oil (French Pharmacopoeia, 8th edition). It is an oily liquid whose properties are presented in Table 1 below.

**Table 1**

| Chemical name | Polyglycolysed oleic glyceride (cores) |
|---|---|
| Trade name | Labrafil M 1944 CS |
| Drop point °C | Liq. |
| Saponification number | 145/175 |
| Acid number | < 2 |
| Iodine number | 60/90 |
| Oral acute toxicity/rat | OLD> 20 ml/kg |
| LOP | 0 |
| HLB | 3/4 |

Lastly, as mono-, di- or triglycerides of natural origin, reference is made in particular to oils of plant origin, such as sweet almond oil, argan oil and palm oil.

According to a preferred embodiment of the present invention, the cellulose polymer is ethyl-cellulose, present in a proportion of between about 1 and 10% by weight, and the oily agent comprises Labrafil® M 1944 CS, present in a proportion of between 5 and 90% by weight, relative to the total weight of the oleogel, the ratio of the weight of oleogel to the weight of aqueous gel being between 10:90 and 90:10. Preferably, the oily agent further comprises propylene glycol isostearate, in a proportion of between 5 and 90% by weight, relative to the total weight of the oleogel.

According to another preferred embodiment of the present invention, the oily agent is chosen from the oily sunscreen filters and more preferably from the cinnamic acid esters. In particular, such an oily agent is chosen from the group consisting of 4-methoxycinnamic acid isopentyl ester, 4- methoxycinnamic ethyl 2-hexyl ester and mixtures thereof. More particularly, the sunscreen filter as the oily agent is 4-methoxycinnamic acid ethyl 2-hexyl ester, such as the product sold under the name "Parsol MCX" by the company Roche.

The oily sunscreen filter may be present in a proportion of about 0.1 to about 20% by weight, and in particular of 4 to 10% by weight, relative to the total weight of the stable gel mixture according to the invention. The corresponding stable gel mixture may be thus used as a sunscreen filter composition per se or as a sunscreen filter composition comprising any further active ingredient such a pharmaceutical or cosmetic active ingredient, as below described.

The aqueous gel present in the stable gel mixture according to the invention comprises at least one gelling agent preferably chosen from carbomers, poloxamers, sodium carboxymethylcellulose and mixtures of the latter, the gelling agent being present in a proportion of between 0.1 and 10% by weight, relative to the total weight of the aqueous gel, the ratio of the weight of oleogel to the weight of aqueous gel formed being between 10:90 and 90:10.

More particularly, the gelling agent for the aqueous gel is the carbomer Carbopol 974, present in a proportion of between 0.1 and 5% by weight, relative to the total weight of the aqueous gel.

Needless to say, the oleogel and the aqueous gel can, respectively, further comprise standard ingredients for a gel, such as texture agents, antioxidants such as butylated hydroxyanisole (BHA) or butylated hydroxytoluene (BHT), dyes or fragrances.

The subject of the present invention is also a process for the preparation of the stable gel mixture as defined above, this process being characterized in that it comprises the steps consisting of:
a) separately forming the oleogel and the aqueous gel, the viscosity of the oleogel formed being adjusted, where necessary by heating preferably to temperature of not more than 50°C, to a value of between 20 and 40 Pa.s, and the viscosity of the aqueous gel formed being adjusted, where necessary by heating preferably to a temperature of not more than 50°C; to a value of between 10 and 30 Pa.s, and
b) incorporating the oleogel into the aqueous gel or, vice versa (i.e. incorporating the aqueous gel to the oleogel), with non-shear stirring, until a mixture of substantially uniform appearance, as already defined above, is obtained.

The respective formations of the oleogel and of the aqueous gel in step a) are carried out according to operating conditions known to those skilled in the art. Reference will be made in particular to the examples below.

According to the invention, the expression "non-shear stirring", for the incorporation of the oleogel into the aqueous gel, or vice versa, in accordance with step b) of the present process, as above described, is understood to refer in particular to a non-destructive stirring of the respective gels of oily and aqueous types, for the formation of a mixture of substantially uniform appearance.

Preferably, a planetary mixer is used, such as those sold by the companies Hobart or Kenwood, set to a maximum speed of about 5 revolutions/minute for about 10 to 20 minutes.

In general, the settings for the other operating conditions in order to carry out the present process are within the scope of those skilled in the art.

The subject of the present invention is also a stable pharmaceutical composition, which can be administered topically, orally or parenterally, characterized in that it comprises a stable gel mixture as defined above, and at least one of the oleogels and the aqueous gels further comprises at least one active ingredient(s).

Needless to say, depending on the route of administration envisaged, the viscosity of the composition is adjusted to an appropriate value, in particular by adapting the proportions of gelling agents in the aqueous gel phase and/or in the oleogel phase.

In particular, given its biodegradable nature, the composition according to the invention can be administered as an injection. It is thus possible to inject allergens or vaccines which are known to be injected in difficult conditions and/or effective only under special administration conditions e.g. in a special solvent that improve antibody formation, such as the Incomplete Freund's Adjuvant (a mixture of an internal aqueous phase dispersed in an external oily phase consisting of mannide monooleate (Arlacel A)). The Incomplete Freund's Adjuvant, however, causes serious inflammatory side effects at the site injection, because it causes a chronic inflammatory response that may be severe and painful depending on the site as well as the quantity and quality of adjuvant injected. The inflammatory response may result in formation of chronic granulomas, sterile abscesses, and/or ulcerating tissue necrosis. In contrast, the oleogel can have the same beneficial properties in aiding the immune response and stimulating antibody formation, but do not have such inflammatory side effects.

Whatever the route of administration envisaged, the active ingredient(s) of the pharmaceutical composition according to the invention can be any pharmaceutically active ingredient(s), such as the above mentioned allergens and vaccines; substances such as hormones which require sustained release while at the same time maintaining good tolerance; or alternatively dermatologically active ingredient(s).

It is also specified that it is possible to use, in the pharmaceutical composition according to the invention, for a topical administration, at least one of the above oily sunscreen filters as the at least one oily agent of the oleogel, provided that the at least one sunscreen filter is not incompatible with any pharmaceutical active ingredient present in the oleogel, i.e. that there is no chemically or therapeutically undesirable reaction between the oily sunscreen filter and the pharmaceutical ingredient.

In addition, the pharmaceutical composition according to the invention can advantageously comprise at least one active ingredient(s) in the oleogel and at least one active ingredient(s) in the aqueous gel, it being possible for at least one active ingredient(s) in the oleogel to be incompatible with at least one active ingredient(s) in the aqueous gel.

According to the invention, the expression "incompatible active ingredient(s)" is understood to refer to active ingredient(s) which are capable of reacting together in a chemically or therapeutically undesirable manner. Insofar as the incompatible active ingredient(s) are mainly soluble, respectively, in the oleogel or the aqueous gel of the composition according to the invention, it has been observed that any contact and thus any adverse reaction between the active ingredient(s) before their administration can thus be avoided.

In particular, the pharmaceutical composition according to the invention can comprise β-oestradiol in the oleogel, in a proportion of between 0.1 and 5% by weight, relative to the total weight of the oleogel, and progesterone in the aqueous gel, in a proportion of between about 1 and 6% by weight, relative to the total weight of the aqueous gel, the ratio of the weight of oleogel to the weight of aqueous gel being between 10:90 and 90:10 and preferably between 30:70 and 70:30.

In certain cases, in particular for the purpose of a topical administration, the pharmaceutical composition according to the invention can comprise the same active ingredient(s) in the oleogel and the aqueous gel.

This is because, in the case of a topical administration, the profile for release of the active ingredient(s) into the skin has been found to be considerably better than that obtained with a composition based on only one type of gel, oily or aqueous, for a given volume of composition applied and a given concentration of active ingredient(s) relative to the total weight of the composition.

This result is particularly advantageous when such a composition is used as an active ingredient(s) reservoir in a transdermal release system as mentioned below. The reason for this is that, on the skin, the amount of active ingredient(s) dissolved in the aqueous gel phase is rapidly taken up by the epidermal layers, which allows the percutaneous passage to start quickly. The amount of active ingredient(s) dissolved in the oleogel phase must firstly, as a function of its oil/water partition coefficient, pass into the aqueous gel phase in order to pass into the epidermal layers : this fraction in the oleogel will thus be released with a greater delay, after the active ingredient(s) dissolved in the aqueous gel phase has been released.

In particular, the pharmaceutical composition according to the invention can advantageously comprise testosterone in the oleogel and the aqueous gel, the proportion of testosterone in the oleogel being between 0.1 and 6% by weight, relative to the total weight of the oleogel, and the proportion of testosterone in the aqueous gel being between 1 and 10% by weight, relative to the total weight of the aqueous gel, and the ratio of the weight of the oleogel to the weight of the aqueous gel being between 10:90 and 90:10 and preferably between 30:70 and 70:30.

Also, the pharmaceutical composition according to the invention can advantageously comprise at least one corticoïd derivative in the oleogel and the aqueous gel, the proportion of the corticoïd derivative in the oleogel being between 0.001 and 3 % by weight, relative to the total weight of the oleogel, and the proportion of the corticoid derivative in the aqueous gel being between 0.001 and 3 % by weight, relative to the total weight of the aqueous gel, and the ratio of the weight of the oleogel to the weight of the aqueous gel being between 5 : 95 and 95 : 5 and preferably between 25 : 75 and 75 : 25 .

According to the invention, the term "corticoïd derivative" is understood to include any structural, natural or synthetic, derivative of the corticoïds, corticoïds being compounds well known from those skilled in the art, including cortisone, 11-oxycorticosteroids, in particular hydroxycortisone.

It has also been observed, surprisingly, that the stable gel mixture according to the invention is particularly suitable for containing a sulphur-containing active ingredient(s), whose characteristic unpleasant odor proves to be advantageously confined in the stable gel mixture and thus cannot be perceived by the user.

Thus, the active ingredient(s) of the pharmaceutical composition according to the invention can be a sulphur-containing active ingredient(s), contained in the aqueous or oleogel.

According to the present invention, the expression sulphur-containing active ingredient(s) is understood to refer in particular to those whose bonding with the sulphur atom is labile, such as the sulphur-containing amino acids and spironolactone.

In particular, the sulphur-containing active ingredient(s) is spironolactone, present in the oleogel in a proportion of between 0.5 and 10% by weight, relative to the total weight of the oleogel, the ratio of the weight of the oleogel to the weight of the aqueous gel being between 10:90 and 90:10 and preferably between 40:60 and 60:40.

The composition according to the invention, as described above, can be prepared by simply adding the active ingcedient(s) during the process for the preparation of the stable gel mixture as already described above. In particular, depending on the pharmaceutical composition envisaged, the active ingredient(s) can be added to the oleogel and/or to the aqueous gel before preparing the oleogel-aqueous gel mixture of substantially uniform appearance. This is the case, in particular, when at least two mutually incompatible active ingredient(s), as defined above, are used, each of the active ingredient(s) being added, one to the oleogel and the other to the aqueous gel. It is also possible to add the active ingredient(s) to the preformed stable gel mixture according to the invention, with stirring, the active ingredient(s) thus being distributed in both the oleogel and the aqueous gel.

Needless to say, a person skilled in the art will know to take into account the characteristics of the active ingredient(s) used, adapting the operating conditions of the process, in particular the temperature, so as possibly not to adversely affect the properties of the active ingredient(s).

The subject of the present invention is also the use of the pharmaceutical composition, as described above, as an active ingredient(s) reservoir in a transdermal release system.

Lastly, the subject of the present invention is also a stable cosmetic composition, characterized in that it comprises a stable gel mixture as defined above, at least one from among the oleogel and the aqueous gel comprising at least one cosmetically active ingredient.

The cosmetically active ingredient can be any ingredient which gives a cosmetic effect, i.e. an ingredient which, when placed in contact with the various external parts of the human body (epidermis, pilous and hair system, nails, lips and outer genital organs) or with the teeth or oral mucosae, by means of the composition, makes it possible, exclusively or mainly, to cleanse them, to fragrance them, to modify their appearance and/or to correct body odors and/or to protect them or keep them in good condition.

The cosmetically active ingredient can thus be, in particular, moisturizers, sunscreens, anti-free-radical agents or skin regenerators which are soluble in either the oleogel or the aqueous gel phases.

Advantageously, given the problems posed by body-care oils in cosmetics, such as bath oils, as explained at the start of the present description, the cosmetically active ingredient is advantageously chosen from the body-care oils, and more particularly from bath oils such as palm oil, sesame oil or argan oil.

It will thus be understood in particular that the oily agent, as defined above, can advantageously constitute a cosmetically active ingredient in the meaning of the invention.

Depending on the type of cosmetically active ingredient used, a person skilled in the art will know how to determine its appropriate proportion by weight, relative to the total weight of the cosmetic composition according to the invention.

The present invention will now be illustrated with the aid of examples, which should not however, under any circumstances, be interpreted as limiting as regards the scope of the invention.

In the text hereinbelow, except where otherwise specified, the percentages indicated are percentages by weight.

### Example 1: preparation of an ethylcellulose + Labrafil M 1944 CS + propylene glycol isostearate + Carbopol 974 gel

### 1.1 Preparation of the oleogel.

The ingredients below are introduced into a Rayneri-type heating mixer, for a mixing temperature of 140°C, the stirring being set at 30 revolutions/minute:

| | |
|---|---|
| - Labrafil® M 1944 CS | 82.5 |
| - Propylene glycol isostearate | 8.5 |
| - Hercules N 50 NF ethylcellulose | 6 |
| - Sesame oil | 1 |
| - Ethanol | 0.5 |
| - BHA | 0.5 |
| - BHT | 0.5 |
| - Fragrance | 0.5 |
| | 100% |

The stirring and the temperature are maintained until a uniform oleogel is obtained. The stirring is then switched off and the mixture is left to cool to room temperature. The oleogel thus obtained has a viscosity of about 60 Pa.s at room temperature.

### 1.2 Preparation of the aqueous gel.

The following ingredients are introduced into another heating mixer, for a mixing temperature of 30°C, with stirring at 30 revolutions/minute:

| | |
|---|---|
| - Carbopol 974 | 0.3 |
| - Water:ethanol mixture (weight ratio 6:5) | 99.7 |
| | 100 % |

The stirring and the temperature are maintained until a uniform aqueous gel is obtained. The stirring is then switched off and the aqueous gel is left to cool to room temperature.

### 1.3 Preparation of the stable gel mixture according to the invention.

The aqueous gel and then the oleogel which are prepared as described in sections 1.1 and 1.2 above are successively introduced into a planetary heating mixer sold by the company Kenwood, set at a speed of 5 revolutions/minute and at a heating temperature of 50°C, the weight ratio of the oleogel to the aqueous gel in the mixture thus formed being 10:90.

The stirring and the temperature are maintained until a gel in the form of a mixture of substantially uniform appearance is obtained. The stirring is then switched off and the mixture is left to cool to room temperature.

The stable gel mixture according to the invention thus prepared has, simultaneously, a thick consistency, a fresh and pleasant feel and surprising stability since no demixing between the oleogel and the aqueous gel was observed on storing the stable gel mixture for a period of more than five years.

Moreover, this stable gel mixture has very good in vitro adhesiveness, which is a parameter that indicates the adhesion of the stable gel mixture to the skin when it is applied thereto.

Measurement of the in vitro adhesiveness was carried out with a "TEC" texturometer as sold by the company ETIA.

The results obtained, in cycles, are as follows:

| | |
|---|---|
| In vitro adhesiveness (mJ) | 0.608 |
| force min (N) | -0.076 |
| Time (sec) | 15.8 |

On account of its excellent in vitro adhesiveness, the stable gel mixture thus obtained can advantageously be used, by incorporating a pharmaceutically active ingredient(s) therein, as a reservoir layer of a system for the transdermal release of the active ingredient(s).

It has also been noticed that a stable gel mixture according to the invention may be prepared as above described but by using a stirring device, for the incorporation of the two oleogel and aqueous gel, providing some shear stirring conditions during the incorporation. It has been more particularly noticed that providing and adjusting such shearing conditions advantageously allows control of the particle size (of each gel ) and /or the viscosity of the resulting stable gel mixture. The one skilled in the art will be able to master such a control possibility by using his common knowledge.

### Example 2: Pharmaceutical composition comprising β-oestradiol and progesterone

The process is performed as in section 1.1 of Example 1, after which an amount of 1% by weight of β-oestradiol, relative to the total weight of the oleogel, is added to the formed oleogel, with stirring and at a temperature set at 50°C, until the β-oestradiol has been uniformly dispersed in the oleogel.

Separately, the process is performed as in section 1.2 of Example 1, after which an amount of 5% by weight of progesterone, relative to the total weight of the aqueous gel, is added to the formed aqueous gel, with stirring and at a temperature set at 50°C, until the progesterone has been uniformly dispersed in the aqueous gel.

The pharmaceutical composition according to the invention is then formed by incorporating the oleogel (containing the β-oestradiol) into the aqueous gel (containing the progesterone) as in section 1.3 of Example 1, the weight ratio of the oleogel to the aqueous gel being 10:90.

### Example 3: Pharmaceutical composition comprising testosterone

The process is performed as in Example 2, using 35 testosterone as active ingredients) instead of β-oestradiol and progesterone. To do this, a proportion of 5% by weight of testosterone, relative to the total weight of the oleogel, is introduced into the oleogel, and, separately, a proportion of 5% by weight of testosterone, relative to the total weight of the aqueous gel, is introduced into the aqueous gel. A weight ratio of oleogel to the aqueous gel of 90: 10 is used.

The testosterone-based pharmaceutical composition thus prepared can advantageously be used as a testosterone-reservoir layer in the form of a gel in a transdermal release system.

### Example 4: Pharmaceutical composition comprising spironolactone

The process is performed as in section 1.1 of Example 1, after which an amount of 5% by weight of spironolactone, relative to the total weight of the oleogel, is added to the formed oleogel, with stirring and at a temperature set at 50°C, until the spironolactone has been uniformly dispersed in the oleogel.

Separately, the process is performed as in section 1.2 of Example 1 in order to form the aqueous gel.

The pharmaceutical composition according to the invention is thus prepared by incorporating the oleogel (containing the spironolactone) to the aqueous gel as in section 1.3 of Example 1, the weight ratio of the oleogel to the aqueous gel being 60:40.

The spironolactone-based pharmaceutical composition according to the invention thus obtained is, surprisingly, substantially free of the unpleasant odor characteristic of the presence of spironolactone, which represents an advantageous deciding factor for users, when compared with the known spironolactone-based pharmaceutical compositions.

## Claims

1. A stable gel mixture, that has a substantially uniform appearance and that comprises at least one oleogel and at least one aqueous gel, the oleogel comprising at least one oily agent gelled with at least one cellulose polymer.

2. The stable gel mixture according to claim 1, wherein the cellulose polymer is chosen from the group consisting of ethylcellulose, non-sodium carboxymethylcellulose and mixtures thereof.

3. The stable gel mixture according to claim 1 or 2, wherein the oily agent is chosen from the group consisting of mono-, di- and triglycerides of synthetic, semisynthetic or natural origin, and mixtures thereof.

4. The stable gel mixture according to claim 3, wherein the oily agent is chosen from the group consisting of a synthetic mono, di- or triglycerides, propylene glycol isostearate, a mixture of polyoxyethylenated oleic glycerides obtained by alcoholysis of natural plant oil and oils of plant origin.

5. The stable gel mixture according to any one of claims 1 to 4, wherein the cellulose polymer is ethyl-cellulose, present in a proportion of between 1% and 10% by weight relative to the total weight of the oleogel; and the oily agent comprises a mixture of polyoxyethylenated oleic glycerides obtained by alcoholysis of natural plant oil, present in a proportion of between 5% and 90% by weight, relative to the total weight of the oleogel; and the ratio of the weight of oleogel to the weight of aqueous gel is between 10:90 and 90:10.

6. The stable gel mixture according to claim 5, wherein the oleogel further comprises propylene glycol isostearate, in a proportion of between 5 % and 90 % by weight, relative to the total weight of the oleogel.

7. The stable gel mixture according to claim 1 or 2, wherein the oily agent is chosen from the oily sunscreen filters.

8. The stable gel mixture according to claim 7, wherein the oily agent is an oily sunscreen filter chosen from the cinnamic acid esters.

9. The stable gel mixture according to any one of claims 1 to 8, wherein the aqueous gel comprises at least one gelling agent selected from the group consisting of carbomers, poloxamers, sodium carboxymethylcellulose and mixtures thereof the gelling agent being present in a proportion of between 0.1 %. and 10 % by weight, relative to the total weight of the aqueous gel, and wherein the ratio of the weight of oleogel to the weight of aqueous gel is between 10:90 and 90:10.

10. The stable gel mixture according to claim 9, wherein the gelling agent is the carbomer Carbopol 974, present in a proportion of between 0.1 % and 5% by weight, relative to the total weight of the aqueous gel.

11. A process for the preparation of the stable gel mixture according to any one of claims 1 to 10, comprising the steps of:
a) forming an oleogel, the viscosity of the oleogel formed being adjusted, where necessary by heating, to a value of between 20 and 40 Pa.s;
b) forming an aqueous gel, the viscosity of the aqueous gel formed being adjusted, where necessary by heating, to a value of between 10 and 30 Pa.s; and
c) incorporating the oleogel formed into the aqueous gel or vice versa, with non-shear stirring, until a stable gel mixture of substantially uniform appearance is obtained.

12. A stable pharmaceutical composition, which can be administered topically, orally or parenterally, comprising a stable gel mixture according to any one of claims 1 to 10, and at least one active ingredient(s) in at least one of the oleogels and the aqueous gels.

13. The pharmaceutical composition according to claim 12, wherein the oleogel comprises at least one active ingredient(s) and the aqueous gel comprises at least one active ingredient(s), it being possible for at least one active ingredient(s) of the oleogel to be incompatible with at least one active ingredient(s) of the aqueous gel.

14. The pharmaceutical composition according to claim 12 or 13, wherein the oleogel comprises β-oestradiol, in a proportion of between 0.1 % and 5% by weight, relative to the total weight of the oleogel; the aqueous gel comprises progesterone, in a proportion of between 1 % and 6 % by weight, relative to the total weight of the aqueous gel; and the ratio of the weight of oleogel to the weight of aqueous gel is between 10:90 and 90:10.

15. The pharmaceutical composition according to claim 12 or 13, wherein the oleogel comprises testosterone, in a proportion of between 0.1% and 6% by weight, relative to the total weight of the oleogel; and the aqueous gel comprises testosterone, in a proportion of between 1% and 10% by weight, relative to the total weight of the aqueous gel; and the ratio of the weight of oleogel to the weight of aqueous gel is between 10:90 and 90:10.

16. The pharmaceutical composition according to claim 12 or 13, wherein the oleogel comprises at least one corticoid derivative, in a proportion of between 0.001% and 3% by weight, relative to the total weight of the oleogel; and the aqueous gel comprises at least one corticoïd derivative, in a proportion of between 0.001% and 3% by weight, relative to the total weight of the aqueous gel; and the ratio of the weight of oleogel to the weight of aqueous gel is between 5:95 and 95:5.

17. The pharmaceutical composition according to claim 12 or 13, wherein the active ingredient is a sulphur-containing active ingredient.

18. The composition according to claim 17, wherein the sulphur-containing active ingredient is spironolactone present in the oleogel in a proportion of between 0.5% and 10% by weight, relative to the total weight of the oleogel, and the ratio of the weight of oleogel to the weight of aqueous gel is between 10:90 and 90:10.

19. Use of the pharmaceutical composition according to any one of claims 12 to 18, for the manufacture of an active ingredient reservoir intented for the use in a transdermal release system.

20. A stable cosmetic composition, comprising the stable gel mixture according to any one of claims 1 to 10 , wherein at least one of the oleogels and the aqueous gels comprise at least one cosmetically active ingredient.

21. The cosmetic composition according to claim 20, wherein the cosmetically active ingredient is chosen from the body-care oils.

## Patentansprüche

1. Stabile Gelmischung, die ein im wesentlichen gleichförmiges Erscheinungsbild aufweist und welche wenigstens ein Oleogel und wenigstens ein wässriges Gel umfasst, wobei das Oleogel wenigstens ein ölartiges Mittel, welches mit wenigstens einem Cellulose-Polymer geliert ist, umfasst.

2. Stabile Gelmischung nach Anspruch 1, wobei das Cellulose-Polymer aus der Gruppe bestehend aus Ethylcellulose, Nicht-Natrium-Carboxymethylcellulose und Mischungen davon ausgewählt wird.

3. Stabile Gelmischung nach Anspruch 1 oder 2, wobei das ölartige Mittel aus der Gruppe bestehend aus Mono-, Di- und Triglyceriden von synthetischem, halbsynthetischem oder natürlichem Ursprung und Mischungen davon ausgewählt wird.

4. Stabile Gelmischung nach Anspruch 3, wobei das ölartige Mittel aus der Gruppe bestehend aus einem synthetischen Mono-, Di- oder Triglyceriden, Propylenglycolisostearat, einer Mischung von polyoxyethylenierten Ölsäureglyceriden, welche durch Alkoholyse von natürlichem pflanzlichem Öl erhalten wird, und Ölen von pflanzlichen Ursprung ausgewählt wird.

5. Stabile Gelmischung nach einem der Ansprüche 1 bis 4, wobei das Cellulose-Polymer Ethylcellulose ist, welche in einem Anteil von zwischen 1 Gew.-% und 10 Gew.-% bezogen auf das Gesamtgewicht des Oleolgels vorhanden ist; und das ölartige Mittel eine Mischung von polyoxyethylenierten Ölsäureglyceriden, welche durch Alkoholyse von natürlichem pflanzlichem Öl erhalten wird, ist, welche in einem Anteil von zwischen 5 Gew.-% und 90 Gew.-% bezogen auf das Gesamtgewicht des Oleolgels vorhanden ist; und das Verhältnis des Gewichts des Oleogels zu dem Gewicht des wässrigen Gels zwischen 10:90 und 90:10 beträgt.

6. Stabile Gelmischung nach Anspruch 5, wobei das Oleogel des weiteren Propylenglycolisostearat in einem Anteil von zwischen 5 Gew.-% und 90 Gew.-% bezogen auf das Gesamtgewicht des Oleolgels umfasst.

7. Stabile Gelmischung nach Anspruch 1 oder 2, wobei das ölartige Mittel aus den ölartigen Sonnenschutzmitteln ausgewählt wird.

8. Stabile Gelmischung nach Anspruch 7, wobei das ölartige Mittel ein ölartiges Sonnenschutzmittel, welches aus den Zimtsäureestern ausgewählt wird, ist.

9. Stabile Gelmischung nach einem der Ansprüche 1 bis 8, wobei das wässrige Gel wenigstens ein Geliermittel, welches aus der Gruppe bestehend aus Carbomeren, Poloxameren, Natriumcarboxymethylcellulose und Mischungen davon ausgewählt wird, umfasst, wobei das Geliermittel in einem Anteil zwischen 0,1 Gew.-% und 10 Gew.-% bezogen auf das Gesamtgewicht des wässrigen Gels vorhanden ist und wobei das Verhältnis des Gewichts des Oleogels zu dem Gewicht des wässrigen Gels zwischen 10:90 und 90:10 beträgt.

10. Stabile Gelmischung nach Anspruch 9, wobei das Geliermittel das Carbomer Carbopol 974 ist, welches in einem Anteil von zwischen 0,1 Gew.-% und 5 Gew.-% bezogen auf das Gesamtgewicht des wässrigen Gels vorhanden ist.

11. Verfahren zur Herstellung der stabilen Gelmischung nach einem der Ansprüche 1 bis 10, welches die Schritte umfasst:
a) ein Oleogel zu bilden, wobei die Viskosität des gebildeten Oleogels, sofern erforderlich, durch Erwärmen auf einen Wert zwischen 20 und 40 Pa.s eingestellt wird;
b) ein wässriges Gel zu bilden, wobei die Viskosität des gebildeten wässrigen Gels, sofern erforderlich, durch Erwärmen auf einen Wert zwischen 10 und 30 Pa.s eingestellt wird; und
c) das gebildete Oleogel in das wässrige Gel einzuarbeiten oder umgekehrt mittels Rühren ohne Scherung, bis eine stabile Gelmischung von im wesentlichen gleichförmigem Erscheinungsbild erhalten wird.

12. Stabile pharmazeutische Zusammensetzung, die topisch, oral oder parenteral verabreicht werden kann, welche eine stabile Gelmischung nach einem der Ansprüche 1 bis 10 und wenigstens einen oder mehrere Wirkstoff(e) in wenigstens einem der Oleogele und der wässrigen Gele umfasst.

13. Pharmazeutische Zusammensetzung nach Anspruch 12, wobei das Oleogel wenigstens einen oder mehrere Wirkstoff(e) umfasst und das wässrige Gel wenigstens einen oder mehrere Wirkstoff(e) umfasst, wobei es möglich ist, dass wenigstens ein oder mehrere Wirkstoff(e) des Oleogels mit wenigstens einem oder mehreren Wirkstoff(en) des wässrigen Gels unverträglich ist.

14. Pharmazeutische Zusammensetzung nach Anspruch 12 oder 13, wobei das Oleogel β-Estradiol in einem Anteil von zwischen 0,1 Gew.-% und 5 Gew.-% bezogen auf das Gesamtgewicht des Oleogels umfasst; das wässrige Gel Progesteron in einem Anteil von zwischen 1 Gew.-% und 6 Gew.-% bezogen auf das Gesamtgewicht des wässrigen Gels umfasst; und das Verhältnis des Gewichts des Oleogels zu dem Gewicht des wässrigen Gels zwischen 10:90 und 90:10 beträgt.

15. Pharmazeutische Zusammensetzung nach Anspruch 12 oder 13, wobei das Oleogel Testosteron in einem Anteil von zwischen 0,1 Gew.-% und 6 Gew.-% bezogen auf das Gesamtgewicht des Oleogels umfasst; und das wässrige Gel Testosteron in einem Anteil von zwischen 1 Gew.-% und 10 Gew.-% bezogen auf das Gesamtgewicht des wässrigen Gels umfasst; und das Verhältnis des Gewichts des Oleogels zu dem Gewicht des wässrigen Gels zwischen 10:90 und 90:10 beträgt.

16. Pharmazeutische Zusammensetzung nach Anspruch 12 oder 13, wobei das Oleogel wenigstens ein Corticoid-Derivat in einem Anteil von zwischen 0,001 Gew.-% und 3 Gew.-% bezogen auf das Gesamtgewicht des Oleogels umfasst; und das wässrige Gel wenigstens ein Corticoid-Derivat in einem Anteil von zwischen 0,001 Gew.-% und 3 Gew.-% bezogen auf das Gesamtgewicht des wässrigen Gels umfasst; und das Verhältnis des Gewichts des Oleogels zu dem Gewicht des wässrigen Gels zwischen 5:95 und 95:5 beträgt.

17. Pharmazeutische Zusammensetzung nach Anspruch 12 oder 13, wobei der Wirkstoff ein schwefelhaltiger Wirkstoff ist.

18. Zusammensetzung nach Anspruch 17, wobei der schwefelhaltige Wirkstoff Spironolacton ist, welches in dem Oleogel in einem Anteil von zwischen 0,5 Gew.-% und 10 Gew.-% bezogen auf das Gesamtgewicht des Oleogels vorhanden ist, und das Verhältnis des Gewichts des Oleogels zu dem Gewicht des wässrigen Gels zwischen 10:90 und 90:10 beträgt.

19. Verwendung der pharmazeutischen Zusammensetzung nach einem der Ansprüche 12 bis 18 für die Herstellung eines Wirkstoffreservoirs, welches für die Verwendung in einem transdermalen Freisetzungssystem bestimmt ist.

20. Stabile kosmetische Zusammensetzung, welche die stabile Gelmischung nach einem der Ansprüche 1 bis 10 umfasst, wobei wenigstens eines der Oleogele und der wässrigen Gele wenigstens einen kosmetisch wirksamen Bestandteil umfassen.

21. Kosmetische Zusammensetzung nach Anspruch 20, wobei der kosmetisch wirksame Bestandteil aus den Körperpflegeölen ausgewählt wird.

## Revendications

1. Mélange de gels stable, qui a un aspect essentiellement uniforme et qui comprend au moins un oléogel et au moins un gel aqueux, l'oléogel comprenant au moins un agent huileux gélifié avec au moins un polymère de cellulose.

2. Mélange de gels stable selon la revendication 1, dans lequel le polymère de cellulose est choisi dans le groupe constitué de l'éthylcellulose, de la carboxy-méthylcellulose non-sodium et de leurs mélanges.

3. Mélange de gels stable selon la revendication 1 ou 2, dans lequel l'agent huileux est choisi dans le groupe constitué des mono-, di- et triglycérides d'origine synthétique, semi-synthétique ou naturelle, et de leurs mélanges.

4. Mélange de gels stable selon la revendication 3, dans lequel l'agent huileux est choisi dans le groupe constitué des mono-, di- ou triglycérides synthétiques, de l'isostéarate de propylèneglycol, d'un mélange de glycérides oléiques polyéthoxylés obtenus par alcoolyse d'huile végétale naturelle et des huiles d'origine végétale.

5. Mélange de gels stable selon l'une quelconque des revendications 1 à 4, dans lequel le polymère de cellulose est l'éthylcellulose, présente en une proportion comprise entre 1 % et 10 % en poids par rapport au poids total de l'oléogel ; et l'agent huileux comprend un mélange de glycérides oléiques polyéthoxylés obtenus par alcoolyse d'huile végétale naturelle, présent en une proportion comprise entre 5 % et 90 % en poids, par rapport au poids total de l'oléogel ; et le rapport du poids d'oléogel par rapport au poids de gel aqueux est entre 10 : 90 et 90 : 10.

6. Mélange de gels stable selon la revendication 5, dans lequel l'oléogel comprend en outre de l'isostéarate de propylèneglycol, en une proportion comprise entre 5 % et 90 % en poids, par rapport au poids total de l'oléogel.

7. Mélange de gels stable selon la revendication 1 ou 2, dans lequel l'agent huileux est choisi parmi les filtres d'écran solaire huileux.

8. Mélange de gels stable selon la revendication 7, dans lequel l'agent huileux est un filtre d'écran solaire huileux choisi parmi les esters d'acide cinnamique.

9. Mélange de gels stable selon l'une quelconque des revendications 1 à 8, dans lequel le gel aqueux comprend au moins un agent gélifiant choisi dans le groupe constitué des carbomères, poloxamères, de la carboxy-méthylcellulose sodium et de leurs mélanges, l'agent gélifiant étant présent en une proportion comprise entre 0,1 % et 10 % en poids, par rapport au poids total du gel aqueux, et dans lequel le rapport du poids d'oléogel au poids de gel aqueux est entre 10 : 90 et 90 : 10.

10. Mélange de gels stable selon la revendication 9, dans lequel l'agent gélifiant est le carbomère Carbopol 974, présent en une proportion comprise entre 0,1 % et 5 % en poids, par rapport au poids total du gel aqueux.

11. Procédé pour la préparation du mélange de gels stable selon l'une quelconque des revendications 1 à 10, comprenant les étapes consistant à :
a) former un oléogel, la viscosité de l'oléogel formé étant ajustée, si nécessaire par chauffage, à une valeur entre 20 et 40 Pa·s ;
b) former un gel aqueux, la viscosité du gel aqueux étant ajustée, si nécessaire par chauffage, à une valeur entre 10 et 30 Pa·s ; et
c) incorporer l'oléogel formé dans le gel aqueux ou réciproquement, avec une agitation sans cisaillement, jusqu'à l'obtention d'un mélange de gels stable d'aspect essentiellement uniforme.

12. Composition pharmaceutique stable, qui peut être administrée par voie topique, orale ou parentérale, comprenant un mélange de gels stable selon l'une quelconque des revendications 1 à 10, et au moins un ou des ingrédients actifs dans au moins l'un des oléogels et des gels aqueux.

13. Composition pharmaceutique selon la revendication 12, dans laquelle l'oléogel comprend au moins un ou des ingrédients actifs et le gel aqueux comprend au moins un ou des ingrédients actifs, avec la possibilité qu'au moins un ou des ingrédients actifs de l'oléogel soient incompatibles avec au moins un ou des ingrédients actifs du gel aqueux.

14. Composition pharmaceutique selon la revendication 12 ou 13, dans laquelle l'oléogel comprend le β-estradiol, en une proportion comprise entre 0,1 % et 5 % en poids, par rapport au poids total de l'oléogel ; le gel aqueux comprend de la progestérone, en une proportion comprise entre 1 % et 6 % en poids, par rapport au poids total du gel aqueux ; et le rapport du poids de l'oléogel par rapport au poids du gel aqueux est entre 10 : 90 et 90 : 10.

15. Composition pharmaceutique selon la revendication 12 ou 13, dans laquelle l'oléogel comprend la testostérone, en une proportion comprise entre 0,1 % et 6 % en poids, par rapport au poids total de l'oléogel ; et le gel aqueux comprend de la testostérone, en une proportion comprise entre 1 % et 10% en poids, par rapport au poids total du gel aqueux ; et le rapport du poids d'oléogel par rapport au poids de gel aqueux est entre 10 : 90 et 90 : 10.

16. Composition pharmaceutique selon la revendication 12 ou 13, dans laquelle l'oléogel comprend au moins un dérivé de corticoïde, en une proportion comprise entre 0,001 % et 3 % en poids, par rapport au poids total de l'oléogel ; et le gel aqueux comprend au moins un dérivé de corticoïde, en une proportion comprise entre 0,001 % et 3 % en poids, par rapport au poids total du gel aqueux ; et le rapport de la masse d'oléogel à la masse de gel aqueux est entre 5 : 95 et 95 : 5.

17. Composition pharmaceutique selon la revendication 12 ou 13, dans laquelle l'ingrédient actif est un ingrédient actif contenant du soufre.

18. Composition selon la revendication 17, dans laquelle l'ingrédient actif contenant du soufre est une spironolactone présente dans l'oléogel en une proportion comprise entre 0,5 % et 10 % en poids, par rapport au poids total de l'oléogel, et le rapport du poids de l'oléogel par rapport au poids du gel aqueux est entre 10 : 90 et 90 : 10.

19. Utilisation de la composition pharmaceutique selon l'une quelconque des revendications 12 à 18 pour la fabrication d'un réservoir d'ingrédient actif destiné à l'utilisation dans un système d'administration transdermique.

20. Composition cosmétique stable, comprenant le mélange de gels stable selon l'une quelconque des revendications 1 à 10, dans laquelle au moins un des oléogels et des gels aqueux comprend au moins un ingrédient actif sur le plan cosmétique.

21. Composition cosmétique selon la revendication 20, dans laquelle l'ingrédient actif sur le plan cosmétique choisi parmi les huiles de soin corporel.
